(19) Europäisches Patentamt
European Patent Office
Office européen des brevets

(11) EP 2 374 456 B1

(12) # EUROPEAN PATENT SPECIFICATION

(45) Date of publication and mention of the grant of the patent:
**03.08.2016 Bulletin 2016/31**

(21) Application number: **09833467.5**

(22) Date of filing: **17.12.2009**

(51) Int Cl.:
*A61K 31/437* (2006.01) *A61P 7/02* (2006.01)
*A61P 9/08* (2006.01) *A61P 9/10* (2006.01)
*A61P 11/00* (2006.01) *C07D 513/04* (2006.01)

(86) International application number:
**PCT/JP2009/071016**

(87) International publication number:
**WO 2010/071164 (24.06.2010 Gazette 2010/25)**

(54) **EDOXABAN DOSAGE REGIME**

EDOXABAN DOSIERUNGSANLEITUNG

POSOLOGIE D'EDOXABANE

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **19.12.2008 JP 2008323578**

(43) Date of publication of application:
**12.10.2011 Bulletin 2011/41**

(73) Proprietor: **Daiichi Sankyo Company, Limited**
**Chuo-ku**
**Tokyo 103-8426 (JP)**

(72) Inventors:
- **ABIKO, Takashi**
  **Tokyo 140-8710 (JP)**
- **UCHIYAMA, Kazuhisa**
  **Tokyo 140-8710 (JP)**
- **MOTOHASHI, Tomoko**
  **Tokyo 140-8710 (JP)**
- **MATSUDA, Tomoko**
  **Tokyo 140-8710 (JP)**
- **SUDA, Miharu**
  **Tokyo 140-8710 (JP)**

(74) Representative: **Wallace, Sheila Jane**
**Marks & Clerk LLP**
**90 Long Acre**
**London WC2E 9RA (GB)**

(56) References cited:
**WO-A1-2008/129846**

- **FURUGOHRI, T. ET AL.: "DU-176b, a potent and orally active factor Xa inhibitor: In vitro and in vivo pharmacological profiles", JOURNAL OF THROMBOSIS AND HAEMOSTASIS, vol. 6, no. 9, 3 June 2008 (2008-06-03), pages 1542-1549, XP002659799,**
- **FURUGOHRI: "Pharmaceutical Characterization, Antithromboti and Bleeding Effects of DU-176b", JOURNAL OF THROMBOSIS AND HAEMOSTASIS, vol. 3 suppl 1, 6 August 2005 (2005-08-06) , - 12 August 2005 (2005-08-12), XP002674667, Abstracts from XXth ISTH Congress**
- **MOHAMMAD UROOJ ZAFAR ET AL: "Antithrombotic effects of factor Xa inhibition with DU-176b: Phase-I study of an oral, direct factor Xa inhibitor using an ex-vivo flow chamber", THROMBOSIS AND HAEMOSTASIS, vol. 98, no. 4, 1 October 2007 (2007-10-01), pages 883-888, XP008151246, SCHATTAUER GMBH, DE; US ISSN: 0340-6245, DOI: 10.1160/TH07-04-0312 [retrieved on 2007-09-10]**
- **Walker M.B.: "Understanding the PT-INR Test", , 2 January 2004 (2004-01-02), XP002674668, Retrieved from the Internet: URL:http://www.vclotacare.com/ptinr.aspx [retrieved on 2012-04-24]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- Anonymous: "A Phase 2, Randomized, Parallel Group, Multi-Center, Multi-National Study for the Evaluation of Safety and Efficacy of Two Fixed Dosages of DU-176b in Subjects With Non-Valvular Atrial Fibrillation", Clinical Trials.gov NCT00806624, 10 December 2008 (2008-12-10), XP002674669, Retrieved from the Internet: URL:http://clinicaltrials.gov/archive/NCT00806624/2008_12_10 [retrieved on 2012-04-23]
- HYLEK ELAINE: "DU-176b, an oral, direct Factor Xa antagonist", CURRENT OPINION IN INVESTIGATIONAL DRUGS, vol. 8, no. 9, 1 September 2007 (2007-09-01), pages 778-783, XP008151244, PHARMAPRESS, US ISSN: 1472-4472
- FURUGOHRI, T. ET AL.: 'DU-176b, a potent and orally active factor Xa inhibitor: In vitro and in vivo pharmacological profiles' JOURNAL OF THROMBOSIS AND HAEMOSTASIS vol. 6, no. 9, 2008, pages 1542 - 1549, XP002659799
- HARUMI TAKAHASHI: '3.Warfarin Oto no Kojinsa' KESSEN TO JUNKAN vol. 14, no. 3, 2006, pages 198 - 202
- MUECK, W. ET AL.: 'Population pharmacokinetics and pharmacodynamics of rivaroxaban - an oral, direct factor Xa inhibitor - in patients undergoing major orthopaedic surgery' CLINICAL PHARMACOKINETICS vol. 47, no. 3, March 2008, pages 203 - 216
- JOHANSSON, L.C. ET AL.: 'Comparison of the Pharmacokinetics and Pharmacodynamics of Ximelagatran in Young and Elderly, Healthy Japanese Men' BLOOD vol. 100, 2002,
- MOULD, D. ET AL.: 'A population pharmacokinetic- Pharmacodynamic and logistic regression analysis of lotrafiban in patients' CLINICAL PHARMACOLOGY AND THERAPEUTICS vol. 69, no. 4, 2001, pages 210 - 222
- RIDOUT, G. ET AL.: 'EFFECT OF RENAL FUNCTION ON EDOXABAN PHARMACOKINETICS (PK) AND ON POPULATION PK/PK-PD MODEL' JOURNAL OF CLINICAL PHARMACOLOGY vol. 49, no. 9, September 2009, page 1124
- MENDELL, J. ET AL.: 'THE PHARMACOKINETICS AND PHARMACODYNAMICS OF THE DIRECT FACTORY XA INHIBITOR, EDOXABAN CO-ADMINISTERED WITH DIGOXIN: A RANDOMIZED, OPEN-LABEL, DUAL TREATMENT SEQUENCE, PARALLEL-GROUP STUDY' JOURNAL OF CLINICAL PHARMACOLOGY vol. 49, no. 9, September 2009, page 1125
- MENDELL, J. ET AL.: 'THOROUGH QT/QTC STUDY WITH EDOXABAN TO EVALUATE EFFECT OF THERAPEUTIC AND SUPRATHERAPEUTIC EXPOSURE ON QTC INTERVAL DURATION IN HEALTHY SUBJECTS' JOURNAL OF CLINICAL PHARMACOLOGY vol. 49, no. 9, September 2009, page 1122

## Description

Technical Field

The present invention relates to an activated blood coagulation factor X (FXa) inhibitor that reduces the risk of bleeding caused by the treatment of thromboembolism in a recipient patient, wherein the dose of the agent can be selected based on a reference value of a dose determinant, specifically creatinine clearance, of the patient in need of administration.

Background Art

$N^1$-(5-chloropyridin-2-yl)-$N^2$-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide represented by the formula (1) shown below or a pharmacologically acceptable salt thereof, or a hydrate thereof, exhibits a strong inhibitory effect directly on activated blood coagulation factor X (FXa), and its usefulness as a preventive drug for thromboembolism, particularly, a prophylactic therapeutic drug for thromboembolism based on deep vein thrombosis (DVT), venous thromboembolism (VTE), or non-valvular atrial fibrillation (NVAF), has been increasingly evident in clinical trials (see e.g., Patent Literature 1 to 4 and Non Patent Literature 1 to 3). The antithrombotic effect of the compound of formula (I) shown below was also evaluated in M. U. Zafar et al, Thrombosis and Haemostasis, Schattauer GmbH, Vol. 98, No. 4, 1 October 2007, pages 883-888. The results showed direct and specific inhibition of FXa by the compound of formula (I) significantly reduced ex-vivo thrombus formation at both venous and arterial rheologies, up to 5 hours post-dose. Furthermore, according to the report, low-molecular FXa inhibitors can be expected to have a therapeutic effect on anticoagulant drug-induced thromboembolism other than thromboembolism based on diseases such as deep vein thrombosis, venous thromboembolism (VTE), or non-valvular atrial fibrillation (NVAF), for example, cerebral infarction, cerebral embolism, myocardial infarction, angina pectoris, pulmonary infarction, pulmonary embolism, acute coronary syndrome (ACS), Buerger's disease, disseminated intravascular coagulation syndrome, thrombosis after prosthetic valve/joint replacement, thrombosis and reocclusion after revascularization, multiple organ dysfunction syndrome (MODS), thrombosis at the time of extracorporeal circulation, or blood coagulation at the time of blood collection (see e.g., Non Patent Literature 1).

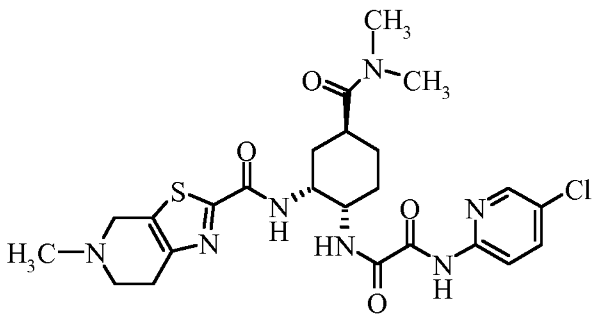

(1)

The increased ability of blood to coagulate is an important factor for diseases such as thromboembolism based on diseases such as unstable angina, cerebral infarction, cerebral embolism, myocardial infarction, pulmonary infarction, pulmonary embolism, acute coronary syndrome (ACS), Buerger's disease, deep vein thrombosis, disseminated intravascular coagulation syndrome, thrombosis after prosthetic valve replacement, reocclusion after revascularization, thrombosis at the time of extracorporeal circulation, venous thromboembolism (VTE), or non-valvular atrial fibrillation (NVAF). Thus, an excellent anticoagulant drug has been demanded, which offers excellent dose response, has prolonged effect, reduces the risk of bleeding, causes less adverse reaction, and immediately produces sufficient effect even by oral administration (see e.g., Non Patent Literature 2).

Heparin in the form of an injection, warfarin in the form of an oral agent, or the like have been used widely as preventive and/or therapeutic drugs for thromboembolism. Direct thrombin receptor inhibitors, direct activated blood coagulation factor X (FXa) inhibitors, and so on have been newly developed in recent years.

Among them, heparins such as heparin, low-molecular heparin, and unfractionated heparin have problems associated with convenience or the like, because they are injections for intravenous administration or injections for subcutaneous administration and are thus administered parenterally. On the other hand, warfarin has such problems that: it takes time to exhibit an effect; its effect varies greatly among individuals of recipient patients; combined use requires caution due to its drug interaction with other agents; it is influenced by food; and it requires monitoring PT-INR (prothrombin time-international normalized ratio) for recipient patients.

Moreover, although anticoagulant therapy using anticoagulants has preventive and/or therapeutic effects on

thromboembolism as described above, it is widely recognized that this therapy has the risk of bleeding as an adverse reaction based on the main pharmacological effect. Thus, it is exceedingly useful to provide an agent that reduces the risk of bleeding.

To reduce the risk of bleeding based on the main pharmacological effect, drugs whose dose is controlled with creatinine clearance (Ccr) or body weight of a patient in need of administration as an index are known such as heparins in the form of injections such as injections for intravenous administration and injections for subcutaneous administration (see e.g., Non Patent Literature 3).

In general, for injections, increase in serum concentration along with increase in dose is linear compared with that for oral agents and varies less among recipient patients. Moreover, the doses of the injections can be easily controlled. Thus, the doses of some injections may be controlled based on a patient-specific factor, for example, creatinine clearance (Ccr) or body weight. On the other hand, most oral agents have great variations in increase in serum concentration along with increase in dose among recipient patients. Thus, because of difficulty in dose control for oral agents, none of the known oral anticoagulant agents reduce the risk of bleeding, with its anticoagulant effect maintained, by controlling the dose of the agent, for example, with a patient individual-specific factor (e.g., creatinine clearance (Ccr)) as an index.

Citation List

Patent Literature

Patent Literature 1: Pamphlet of International Publication No. WO 2003/000680
Patent Literature 2: Pamphlet of International Publication No. 2004/058715

Non Patent Literature

Non Patent Literature 1: Thrombosis Research, Vol. 15, p. 617-629, 1979
Non Patent Literature 2: Thrombosis Research, Vol. 68, p. 507-512, 1992
Non Patent Literature 3: PRODUCT INFORMATION, CLEXANE ® AND CLEXANE ® FORTE, CLEXANE ® PI MKT, #6178v16, Pages 1-19

Summary of Invention

Technical Problem

An object of the present invention is to provide an activated blood coagulation factor X (FXa) inhibitor that reduces the risk of bleeding caused by the treatment of thromboembolism.

Solution to Problem

The present inventors have completed the present invention by finding that an oral anticoagulant agent comprising $N^1$-(5-chloropyridin-2-yl)-$N^2$-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide (1) or a pharmacologically acceptable salt thereof, or a hydrate thereof, as an active ingredient can reduce the risk of bleeding caused by the treatment of thromboembolism by selecting its dose with a measured value of a dose determinant, specifically creatinine clearance, of a patient in need of administration as an index.

Specifically, the present invention relates to:

(1) an oral anticoagulant agent comprising $N^1$-(5-chloropyridin-2-yl)-$N^2$-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide represented by the following formula (1):

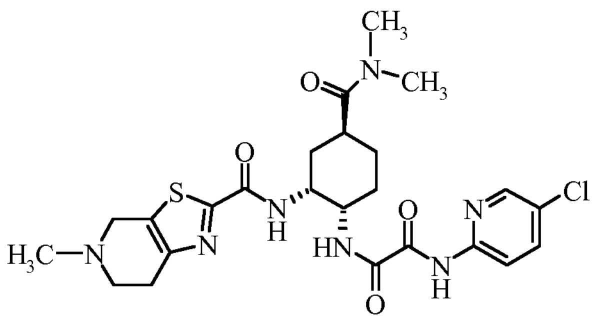

(1)

or a pharmacologically acceptable salt thereof, or a hydrate thereof, as an active ingredient, for use in the treatment of thromboembolism based on non-valvular atrial fibrillation wherein the dose of the anticoagulant agent is selected by

(A) selecting a factor involved in the risk of bleeding caused by the anticoagulant agent as a dose determinant, wherein the dose determinant of a patient in need of administration is creatinine clearance;
(B) setting a reference value of the dose determinant, wherein the reference value of the creatinine clearance is 50ml/min;
(C) measuring the dose determinant of a patient in need of administration; and
(D) selecting the dose of the anticoagulant agent such that: (i) when the measured value is larger than the reference value, the dose is 30 to 60 mg/day in terms of the amount of the compound of formula (1); or (ii) when the measured value is equal to or smaller than the reference value, the dose is 1/2 that in the case where the measured value is larger than the reference value.

Advantageous Effects of Invention

According to the present invention, an oral anticoagulant agent comprising $N^1$-(5-chloropyridin-2-yl)-$N^2$-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide (1) or a pharmacologically acceptable salt thereof, or a hydrate thereof, as an active ingredient is provided as an agent that is effective for the treatment of thromboembolism and has an inhibitory effect on activated blood coagulation factor X (FXa), with a reduced risk of bleeding.

Brief Description of Drawings

[Figure 1] Figure 1 is a table showing the dose of a compound (1a) (5 mg to 60 mg [each dose is based on the amount of a compound (1) in a free form]) and its preventive effect on venous thromboembolism (VTE).
[Figure 2] Figure 2 is a diagram showing the dose of the compound (1a) (5 mg to 60 mg [each dose is based on the amount of the compound (1) in a free form]) and its preventive effect on venous thromboembolism (VTE). The inhibitory effect shown in [Figure 1] is represented graphically in the diagram.
[Figure 3] Figure 3 is a diagram showing the dose of the compound (1a) (5 mg to 60 mg [each dose is based on the amount of the compound (1) in a free form]) and the incidence of bleeding events.
[Figure 4] Figure 4 is a diagram summarizing patient backgrounds such as the ages, body weights, and risk factors of test subjects in groups to which 30 mg compound (1a), 45 mg compound (1a), or 60 mg compound (1a) [each dose is based on the amount of the compound (1) in a free form] had been administered, and a group to which warfarin had been administered .
[Figure 5] Figure 5 is a diagram showing, in the form of a graph, the incidence of bleeding events in each of the groups to which 30 mg compound (1a), 45 mg compound (1a), or 60 mg compound (1a) [each dose is based on the amount of the compound (1) in a free form] had been administered, and the group to which warfarin had been administered.
[Figure 6] Figure 6 is a diagram showing, in the form of a graph, the incidence of bleeding events in two groups to which compound (1a) had been administered divided according to the creatinine clearances (Ccr) of the test subjects (Ccr≤50; and Ccr>50) and in a group to which warfarin had been administered (Warfarin).
[Figure 7] Figure 7 is a diagram showing the distribution of body weight (BW; abscissa) vs. creatinine clearance (Ccr; ordinate) of test subjects that received the compound (1a), wherein the filled squares represent test subjects that exhibited bleeding events.

[Figure 8] Figure 8 is a diagram showing the median values and widths of the incidence of bleeding events in each of four groups [(1) to (4)] divided according to (1) BW>60 kg, Ccr≤50; (2) BW>60 kg, Ccr>50; (3) BW≤60 kg, Ccr>50; and (4) BW≤60 kg, Ccr≤50 from the distribution diagram of [Figure 7].

Description of Embodiments

Hereinafter, the present invention will be described in detail.

In the present specification, a specific example of an "activated blood factor X (FXa) inhibitor" is preferably a compound represented by the formula (1) [hereinafter, abbreviated to a compound (1)]. The compound (1) may be a free form (free base), a hydrate thereof, a pharmacologically acceptable salt thereof, or a hydrate of the salt.

Examples of the pharmacologically acceptable salt of the compound represented by the formula (1) include hydrochloride, sulfate, hydrobromide, hydroiodide, phosphate, nitrate, benzoate, methanesulfonate, 2-hydroxyethanesulfonate, p-toluenesulfonate, acetate, propionate, oxalate, malonate, succinate, glutarate, adipate, tartrate, maleate, fumarate, malate, and mandelate.

The salt of the compound represented by the formula (1) is preferably hydrochloride or p-toluenesulfonate, particularly preferably p-toluenesulfonate.

Preferred examples of the compound represented by the formula (1) can include the following: $N^1$-(5-chloropyridin-2-yl)-$N^2$-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide; $N^1$-(5-chloropyridin-2-yl)-$N^2$-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide hydrochloride; $N^1$-(5-chloropyridin-2-yl)-$N^2$-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide p-toluenesulfonate; and $N^1$-(5-chloropyridin-2-yl)-$N^2$-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide p-toluenesulfonate monohydrate.

Among these preferred compounds, $N^1$-(5-chloropyridin-2-yl)-$N^2$-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide p-toluenesulfonate monohydrate represented by the following formula (1a) (compound 1a) :

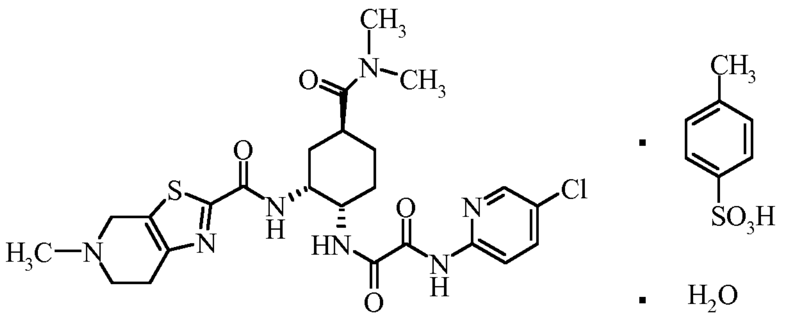

(1a)

is particularly preferred.

The free base (free form) of the "activated blood coagulation factor X (FXa) inhibitor" means the salt (acid-addition salt) and/or the hydrate formed with the "activated blood coagulation factor X (FXa) inhibitor" except for the "acid" in the acid-addition salt or the "water" in the hydrate. For example, the free base (free form) of the compound (1a) means $N^1$-(5-chloropyridin-2-yl)-$N^2$-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide (1) represented by the following formula (1):

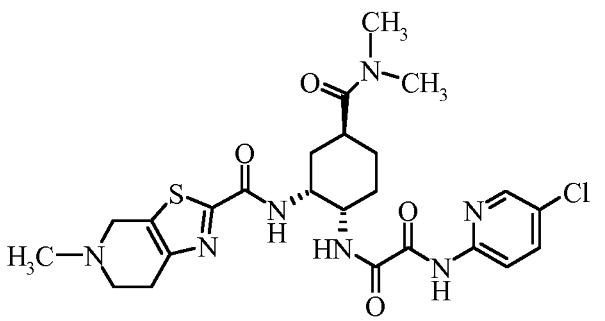

(1)

In the present specification, all the numeric values of doses used are doses in terms of the amount of the compound (1) as a free base (free form).

The dose of the $N^1$-(5-chloropyridin-2-yl)-$N^2$-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide p-toluenesulfonate monohydrate (compound 1a) serving as an activated blood coagulation factor X (FXa) inhibitor is preferably 5 mg to 90 mg, more preferably 15 mg to 60 mg, in terms of the amount of the free base (free form) [compound (1) described above].

When administered as an oral anticoagulant agent, the $N^1$-(5-chloropyridin-2-yl)-$N^2$-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide or the salt thereof, or the hydrate thereof, serving as an activated blood coagulation factor X (FXa) inhibitor is administered to a patient, for example, in a dosage form such as a tablet together with pharmacologically acceptable additives. The content of the free base (free form) [compound (1) described above] of the $N^1$-(5-chloropyridin-2-yl)-$N^2$-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide serving as an activated blood coagulation factor X (FXa) inhibitor, contained in the administered preparation, is preferably 15 mg, 30 mg, 45 mg, or 60 mg, particularly preferably 15 mg, 30 mg, or 60 mg. The tablet containing 15 mg, 30 mg, 45 mg, or 60 mg of the compound (1) may be provided in its shape with a score line for easily dividing equally the active ingredient in the tablet. For example, the scored tablets (amount of the compound (1): 60 mg and 30 mg) can be divided at the score line into tablets containing 30 mg and 15 mg of the compound (1), respectively. For tablets corresponding to 45 mg of the compound (1), the tablet containing 30 mg of the compound (1) can be divided at the score line into a tablet containing 15 mg of the compound (1), which is in turn combined with another tablet containing 30 mg of the compound (1) to provide the desired dose. Thus, it is not required that all of the tablets with the preferred contents should be prepared separately for each dose. Thus, particularly preferred examples of the content of the compound (1) can include 60 and 30 mg/tablet. These tablets containing 60 mg and 30 mg of the compound (1) preferably have a score line for equal division.

In the present specification, creatinine clearance (Ccr) is represented by the following equation 1:

$$Ccr\,(ml\,/\,min) = \frac{U \times V}{S} \times \frac{1.73}{A} \qquad \text{(Equation 1)}$$

U: urinary creatinine concentration (mg/dl);
V: urine volume in 1 minute (ml/min);
S: serum creatinine concentration (mg/dl);
A: body surface area ($m^2$);
1.73: average body surface area of Japanese ($m^2$) (in the year 2001 meeting of the Japanese Society of Nephrology, the average body surface area of Japanese was changed from 1.48 $m^2$ conventionally used to 1.73 $m^2$.)

The reference range of the creatinine clearance (Ccr) differs depending on age and sex. Particularly, the creatinine clearance significantly decreases with aging, and its normal level is allegedly 100 ml/min or higher on average for men and women.

Moreover, for the body surface area [BSA ($m^2$)] used in equation 1, the so-called "Du Bois formula" represented by the following equation 2:

$$\text{Body surface area} = [BW]^{0.425} \times [HT]^{0.725} \times 0.007184 \quad \text{(Equation 2)}$$

[HT]: height (cm)
[BW]: body weight (kg)

has been reported as a calculation formula of the body surface area [BSA ($m^2$)] of Americans and Europeans (Du Bois, Arch Int Med, 17: 863, 1916). The world standard D "Du Bois formula" is preferably used for the body surface area in chemotherapy.

A calculation formula represented by the following equation 3:

$$\text{Body surface area} = [BW]^{0.425} \times [HT]^{0.725} \times 0.007358 \quad \text{(Equation 3)}$$

[HT]: height (cm)
[BW]: body weight (kg)

or the following equation 4:

$$\text{Body surface area}=[BW]^{0.663}\times[HT]^{0.444}\times 0.008883 \quad \text{(Equation 4)}$$

[HT]: height (cm)
[BW]: body weight (kg)

(Fujimoto et al., Japanese Journal of Nutrition, Vol. 28, No. 6, 1968) is known as an equation suitable for Japanese.

The creatinine clearance (Ccr) can be easily calculated from a urinary creatinine concentration (mg/dl), a urine volume in 1 minute (ml/min), a serum creatinine concentration (mg/dl), and a body surface area ($m^2$), as described above.

In the present specification, a dose determinant for the administered agent means creatinine clearance (Ccr). The reference value for determining the dose of the administered agent is: for creatinine clearance (Ccr), preferably 50 ml/min.

In the present specification, the "dose determinant" means that: using creatinine clearance (Ccr) as the dose determinant, dose reduction can be selected, for example, when the Ccr of a patient is equal to or smaller than the reference value 50 ml/min (and is 30 ml/min or larger). In this context, for the reduction, reduction by 1/2 is selected.

Since creatinine clearance (Ccr) serving as the dose determinant is a variable, the dose may be reduced by 1/2, or reset to the starting dose from 1/2, or kept in this reduced amount, depending on the variation in the dose determinant.

In the present specification, examples of preferable aspects of the reduction based on the dose determinant can include those described below. For example, it is preferred to select dose reduction by 1/2 when the creatinine clearance (Ccr) serving as the dose determinant decreases to 50 ml/min or smaller [and 30 ml/min or larger (≥30 ml/min to ≤50 ml/min)]. In this case, it is more preferred to select the reduction by 1/2 when: after 1 week or later from this decrease of the dose determinant Ccr to 50 ml/min or smaller [and 30 ml/min or larger (≥30 ml/min to ≤50 ml/min)], the Ccr is remeasured and confirmed to be 50 ml/min or smaller [and 30 ml/min or larger (≥30 ml/min to ≤50 ml/min)]; and the decrease of the Ccr is 20% or more compared to the Ccr at the start of administration of the agent (initial Ccr). Furthermore, even when the dose determinant Ccr exceeds 50 ml/min after the reduction by 1/2, the dose may be kept in this reduced amount.

In the present specification, for selecting appropriate dose reduction based on the reference value of the dose determinant, creatinine clearance (Ccr) is measured and the dose reduced to 1/2 when the factor becomes equal to or lower than the reference value.

In the present specification, for adjusting the dose based on the "reference value of the dose determinant", reduction by 1/2 is selected. A more specific aspect of the phrase " based on a measured value of a dose determinant of a patient in need of administration, the dose can be selected with the reference value of the dose determinant as an index" is preferably (1) dose reduction from 60 mg to 30 mg or (2) dose reduction from 30 mg to 15 mg.

In the present specification, examples of disease attributed to blood coagulation include thrombosis and/or embolism, and these are widely recognized as thromboembolism.

In the present specification, examples of thromboembolism can include, but are not limited to: one or more diseases selected from the group consisting of the following:

"cerebral infarction, cerebral embolism, myocardial infarction, angina pectoris, pulmonary infarction, pulmonary embolism, acute coronary syndrome (ACS),
Buerger's disease, deep vein thrombosis, disseminated intravascular coagulation syndrome, thrombosis after prosthetic valve/joint replacement, thrombosis and
reocclusion after revascularization, multiple organ dysfunction syndrome (MODS), thrombosis at the time of extracorporeal circulation, blood coagulation at the time of blood collection, venous thromboembolism (VTE), and non-valvular atrial fibrillation (NVAF)",
or thromboembolism based on the disease(s).

The thromboembolism is preferably thromboembolism based on cerebral infarction, cerebral embolism, myocardial infarction, angina pectoris, pulmonary infarction, pulmonary embolism, acute coronary syndrome (ACS), Buerger's disease, deep vein thrombosis, disseminated intravascular coagulation syndrome, thrombosis after prosthetic valve/joint

replacement, thrombosis and reocclusion after revascularization, multiple organ dysfunction syndrome (MODS), thrombosis at the time of extracorporeal circulation, blood coagulation at the time of blood collection, venous thromboembolism (VTE), or non-valvular atrial fibrillation (NVAF), more preferably thromboembolism based on venous thromboembolism (VTE) or non-valvular atrial fibrillation (NVAF).

In the present specification, examples of markers serving as an index in the test of thromboembolism can include D-dimer, F1+2, TAT, soluble fibrin, and PIC in serum. For example, D-dimer is a degradation product formed in the course of the thrombolytic process and is a marker whose level rises during secondary fibrinolysis in thrombosis. TAT and F1+2 are markers of thrombin formation and serve as an index for coagulation activity. Moreover, soluble fibrin is a marker of fibrin formation and seems likely more to reflect increased ability to coagulate. PIC serves as an index for potentiated fibrinolytic systems. Anti-Xa activity is also effective for confirming the main pharmacological effect.

The duration of administration of the agent differs depending on the disease and is, for example, a relatively short period of 2 to 4 weeks in the orthopaedic field such as the prevention and/or treatment of thromboembolism after joint replacement surgery, or a relative long period of 1 month or longer in the internal medicine field such as the prevention and/or treatment of thromboembolism based on chronic disease, for example, cerebral infarction, cerebral embolism, myocardial infarction, angina pectoris, pulmonary infarction, pulmonary embolism, venous thromboembolism, acute coronary syndrome (ACS), Buerger's disease, deep vein thrombosis, disseminated intravascular coagulation syndrome, multiple organ dysfunction syndrome (MODS), thrombosis at the time of extracorporeal circulation, blood coagulation at the time of blood collection, venous thromboembolism (VTE), or non-valvular atrial fibrillation (NVAF).

In the present specification, a short period means 2 to 4 weeks, preferably, within 2 weeks, as the duration of administration of the agent. Moreover, a long period means a period exceeding 20 days, preferably a period exceeding 4 weeks, as the duration of administration of the agent.

In the present specification, the term "bleeding" in the risk of bleeding abides by the following definitions (1) to (3):

(1) major bleeding: (A) fatal bleeding, (B) clinically evident bleeding accompanied by decrease in hemoglobin level exceeding 2 g/dL, (C) clinically evident bleeding that requires transfusion (except for autologous transfusion with donated autologous blood) exceeding 4 units (1 unit: approximately 200 cc), (D) retroperitoneal bleeding, intracranial bleeding, bleeding in the eye, or bleeding in the bone-marrow cavity, and (E) bleeding that requires reoperation;
(2) clinically relevant non-major bleeding: the following bleeding events that do not apply to major bleeding are defined as clinically relevant non-major bleeding: (A) hematoma of 5 cm or larger in the major axis, (B) nosebleed or gingival bleeding that manifests itself without external factors and continues for 5 minutes or longer, (C) gastrointestinal bleeding, (D) macroscopic hematuria that lasts more than 24 hours, and (E) other bleeding events that are judged as being clinically relevant non-major bleeding by a principal investigator or sub-investigator; and
(3) minor bleeding: all bleeding events except for major bleeding or clinically relevant non-major bleeding.

In the present specification, QD, an abbreviation of "Quaque Die", represents the number of doses of the agent and means that the agent is administered once a day.

In the present specification, the preparation means an oral preparation and is administered to a patient in dosage forms such as tablets, granules, powders, capsules, or syrups comprising the active ingredient and pharmacologically acceptable additives. Examples of the pharmacologically acceptable additives can include excipients, disintegrants, binders, fluidizers, lubricants, coloring agents, and lustering agents.

In the present specification, generally used significance tests and data analysis approaches can be used. Generally used commercially available statistical software or the like may be used in analyses.

Examples

Next, the present invention will be described in detail with reference to Examples. However, the present invention is not intended to be by any means limited to these Examples.

(Example 1)

Patients that underwent total knee arthroplasty were used as test subjects in a randomized double-blind dose comparison study with a placebo as a control to verify their dose responses to the preventive effect (efficacy) of a compound (1a) on deep vein thrombosis (DVT) and pulmonary embolism (PE) and examine the safety of the compound (1a).

For doses and an administration method, administration was initiated 6 to 24 hours after the operation and performed in the morning as a rule from the day following the first administration. A preparation containing the compound (1a) as an active ingredient was orally administered as an investigational new drug once a day for 11 to 14 days.

Administration groups were divided into a total of 5 groups: a placebo group and compound (1a) groups (5 mg,

15 mg, 30 mg, and 60 mg [each dose is based on the amount of a compound (1) in a free form]).

The clinical trial was conducted by a method that involved, after obtaining the consent of the clinical trial participants, conducting preliminary tests within 14 days before the operation, initiating drug or placebo administration within 6 to 24 hours after the operation, and performing the administration for 11 to 14 days. Venography was performed within 24 hours after the completion of administration, and follow-up tests were further conducted 25 to 35 days after the completion of administration.

The efficacy was evaluated using, as primary endpoints, the frequencies of cases that exhibited one or more of the following thromboembolic events observed from the initiation of administration of the investigational new drug to the implementation of venography after the completion of administration of the investigational new drug:

(1) DVT (DVT evaluated by venography on both lower limbs at the completion of administration of the investigational new drug), (2) definitely diagnosed symptomatic PE, (3) symptomatic DVT confirmed before the predetermined venography, and (4) VTE (DVT or PE).

The safety was evaluated using the incidence of major bleeding, clinically relevant non-major bleeding, and minor bleeding.

Biomarkers were evaluated on (1) anti-Xa activity, (2) D-dimer, (3) F1+2, (4) TAT, (5) soluble fibrin, and (6) PIC. These biomarkers were selected for exploratively studying efficacy. D-dimer was selected because it is an index that reflects secondary fibrinolysis. TAT and F1+2 were selected because they are markers of thrombin formation and serve as an index for coagulation activity. Moreover, soluble fibrin, which has become measurable in recent years, was selected because it is a marker of fibrin formation and seems likely more to reflect increased ability to coagulate. PIC was selected because it serves as an index for potentiated fibrinolysis systems. Anti-Xa activity was selected to confirm the main pharmacological effect.

Main statistical analysis was conducted on the incidence of thromboembolic events using the Cochran-Armitage test to verify dose response. Moreover, the difference between the placebo group and each compound (1a) group and its 95% confidence interval were calculated, and the placebo group and each compound (1a) group were compared using the procedures of the Shirley-Williams test. Moreover, the difference between the placebo group and each compound (1a) group and its 95% confidence interval were calculated on the incidence of major bleeding and the incidence of clinically relevant non-major bleeding, and 2 groups (the placebo group and each compound (1a) group) were compared using the $X^2$ (chi$^2$) test.

In the clinical trial, the compound (1a) was evaluated for its therapeutic effect in a late phase 2 clinical trial (deep vein thrombosis) (randomized double-blind placebo-controlled dose comparison test with total knee arthroplasty patients as test subjects). This clinical trial was carried out in conformity with the standards specified by Article 14 Paragraph 3 and Article 80-2 of the Pharmaceutical Affairs Law and MHW Ordinance No. 28 "Standards for the Implementation of Clinical Trials on Pharmaceutical Products" (March 27, 1997; hereinafter, referred to as the GCP ordinance). The implementation of this clinical trial conformed to the ethical research guidelines of the Declaration of Helsinki and secured the maximal human rights, welfare, and safety of the test subjects. Moreover, this clinical trial was examined by the institutional review board of a medical institution implementing it or the institutional review board specified by Article 27 of the GCP ordinance and carried out after getting the approval, and whether or not the clinical trial was continued was examined at a once-a-year frequency or at a frequency requested by the institutional review board or more [Protocol No. of this clinical trial: document information "PRT_CP2P3v1.0J"]

<Test Results>

The ages, body weights, and so on of the test subjects did not differ largely between each group to which compound (1a) had been administered and the group to which placebo had been administered.

As shown in [Figure 1] and [Figure 2], the compound (1a) exhibited an inhibitory effect of 39 to 81% on asymptomatic venous thromboembolism and symptomatic venous thromboembolism in a manner dependent on its dose of 5 mg to 60 mg [each dose is based on the amount of the compound (1) in a free form]. This inhibitory effect was significant ($P \leq 0.005$)) compared to the inhibition of placebo administration. Moreover, as shown in [Figure 3], the number of bleeding events was increased in a manner dependent on the dose of the compound (1a) (5 mg to 60 mg). However, the incidence of these bleeding events did not differ from that in the group to which the placebo had been administered. Moreover, as seen from the serum biochemistry test results, the group to which compound (1a) had been administered and the group to which placebo had been administered have no difference in liver functions.

Example 2

Non-valvular atrial fibrillation patients were used as test subjects to compare the incidence of bleeding events

among groups to which compound (1a) had been administered with warfarin potassium (hereinafter, referred to as warfarin) as a control. Moreover, secondary evaluations were performed for efficacy by the comparison of the incidence of thromboembolic events, pharmacodynamic indexes, and biomarkers, and for safety by the comparison of the incidences of adverse events and adverse reactions. The clinical trial is a multicentre randomized dose comparison study, which was conducted as a double-blind test for the groups to which compound (1a) had been administered and as an open-label test for the group to which warfarin had been administered. The control drug warfarin was evaluated in an open-label manner due to difficult dose adjustment, although its efficacy on embolism in non-valvular atrial fibrillation patients has been demonstrated. Thus, only the groups to which compound (1a) had been administered were evaluated by a double-blind test among doses. Moreover, the drug was orally administered once a day for 12 weeks as a rule to a total of 4 groups: 3 groups to which compound (1a) was administered (30 mg, 45 mg, and 60 mg [each dose is based on the amount of the compound (1) in a free form]) and a group to which warfarin was administered. In the group to which warfarin was administered, 0.5-mg and 1-mg warfarin tablets were used and orally administered once a day for 12 weeks as a rule at a dose that allowed PT-INR to be controlled to between 2.0 and 3.0 inclusive (for 70-year-old or older participants, between 1.6 and 2.6 inclusive).

The clinical trial was carried out by, after obtaining the consent of test subjects, conducting head diagnostic imaging before registration to confirm no bleeding observation, and conducting head diagnostic imaging (CT or MRI), transthoracic echocardiography, lead electrocardiography, blood pressure examination, pulse rate examination, and hematological/biochemical tests. Moreover, blood collection from the groups to which compound (1a) had been administered for the assay of pharmacodynamic indexes and biomarkers shown below was conducted as median measurement before the initiation of administration, on the 4th week into the administration of the investigational new drug in the treatment period [before taking the investigational new drug (trough) and 1 to 3 hours after taking the investigational new drug], on the 8th week thereinto [before taking the investigational new drug (trough)], on the 12th week thereinto [at the visit at hospital or at the time of discontinuation (if possible)], and at the visit at hospital on the 4th and 8th weeks after the completion of administration of the investigational new drug in the treatment period or after discontinuation. On the other hand, blood collection from the group to which warfarin had been administered for the assay of pharmacodynamic indexes and biomarkers shown below was conducted before the initiation of administration, at the visit at hospital on the 4th, 8th, and 12th weeks into administration of the investigational new drug in the treatment period or at the time of discontinuation (if possible), and at the visit at hospital on the 4th and 8th weeks after the completion of administration of the investigational new drug in the treatment period or after discontinuation [pharmacodynamic indexes: PT, PT-INR, and APTT]; [biomarkers: F1+2, TAT, and D-dimer].

The incidence of bleeding events [major bleeding (Major), clinically relevant non-major bleeding (Clinically Relevant), and minor bleeding (Minor)] observed during the treatment period was examined as primary endpoints.

Moreover, 1) the incidence of thromboembolic events, 2) the incidences of adverse events and adverse reactions, and 3) the incidences of adverse events and adverse reactions observed during the treatment period were examined as secondary endpoints.

The pharmacodynamic indexes were selected for examining anticoagulant ability for atrial fibrillation patients.

The biomarkers F1+2 and TAT are markers of thrombin formation, while D-dimer is a marker of secondary fibrinolysis. According to the report, their levels are increased in atrial fibrillation patients and decreased by anticoagulant therapy using warfarin (N. Vene et al., Thromb Haemost 2003; 90: 1163-72). Moreover, the compound (1a) has been confirmed in early phase II trials to reduce the levels of these biomarkers through its administration. Thus, the influences of the compound (1a) and warfarin on these biomarkers were studied in this trial.

(A) the plasma concentration of the compound (1), (B) the pharmacokinetic parameters of the population, (C) the pharmacokinetic parameters of each test subject, and (D) the correlation between the pharmacokinetic parameters of each test subject and the occurrence of bleeding events were evaluated as pharmacokinetic endpoints. These endpoints were selected on the basis of the purpose of elucidating pharmacokinetic properties in the atrial fibrillation patient population. Moreover, the correlation between bleeding events and pharmacokinetic parameters was selected for use as a reference for determining the clinically recommended dose of the compound (1a).

The primary endpoints were analyzed by a method that involved in the main analysis, calculating the difference in the incidence of bleeding events between the group to which warfarin had been administered and each group to which compound (1a) had been administered and its 95% confidence interval using the Score method (including hemoglobin less than 10 g/dL or the number of platelets less than $10 \times 10^4/\mu L$ in preliminary tests) For each administered group, the incidence of bleeding events and its 95% confidence interval were also calculated using the Score method [including: the presence of active bleeding at the time of registration; subcutaneous bleeding with one or more observed hematomas of 5 cm or larger in the major axis; macroscopic hematuria, occult blood in the urine with 2+, occult blood in the urine with + and erythrocyte sedimentation of 10 cells/HPF or larger in the urine observed in urine tests (except for occult blood in the urine with 2+ continuously observed since 1 year ago from the consent date)]. Furthermore, 2 groups were compared among the compound (1a) groups using the $X^2$ ($chi^2$) test.

In the secondary analysis, the hypothesis (comparison) shown below was studied on the incidence of bleeding

events using the one-sided Cochran-Armitage test at the 0.025 level of significance. The secondary endpoints were analyzed by calculating the difference in the incidence of bleeding events in patients at risk of having non-valvular atrial fibrillation (NVAF) between the warfarin group and each compound (1a) group and its 95% confidence interval. Moreover, the dose response of the compound (1a) group was studied using the Cochran-Armitage test.

This clinical trial was carried out in conformity to the standards specified by Article 14 Paragraph 3 and Article 80-2 of the Pharmaceutical Affairs Law and MHW Ordinance No. 28 "Standards for the Implementation of Clinical Trials on Pharmaceutical Products" (March 27, 1997; hereinafter, referred to as the GCP ordinance). The implementation of this clinical trial conformed to the ethical research guidelines of the Declaration of Helsinki and secured the maximal human rights, welfare, and safety of the test subjects. [Protocol No. of this clinical trial: DU-176b-C-J225_Ver.03.05.000].

<Test Results>

As shown in [Figure 4], the ages, body weights, risk factors, and so on of the test subjects did not differ largely between the groups.

As shown in [Figure 5], the incidence of bleeding events [major bleeding (Major), clinically relevant non-major bleeding (Clinically Relevant), and minor bleeding (Minor)] was increased as the dose of the compound (1a) got higher. This incidence had no significant difference from that in the warfarin group (Warfarin).

[Figure 6] is a diagram showing, in the form of a graph, the incidence of bleeding events in two groups to which compound (1a) had been administered divided according to the creatinine clearances (Ccr) of the test subjects (Ccr≤50; and Ccr>50) and in a group to which warfarin had been administered (Warfarin). As shown in [Figure 6], the incidence of bleeding events was higher in the group to which compound (1a) had been administered involving the test subjects having creatinine clearance (Ccr) of Ccr≤50 than in the corresponding group involving the test subjects having Ccr>50 by the administration at 45 mg/kg or higher to the patient groups.

[Figure 7] is a diagram showing the distribution of body weight (BW; abscissa) vs. creatinine clearance (Ccr; ordinate) of test subjects that received the compound (1a), wherein the filled squares represent test subjects that exhibited bleeding events. [Figure 8] is a diagram showing the median values and widths of the incidence of bleeding events in each of four groups [(1) to (4)] divided according to (1) BW>60 kg, Ccr≤50; (2) BW>60 kg, Ccr>50; (3) BW≤60 kg, Ccr>50; and (4) BW≤60 kg, Ccr≤50 from the distribution diagram of [Figure 9]. As a result, the highest incidence of bleeding events was obtained in the group of (4) BW≤60 kg, Ccr≤50, and the incidence of bleeding events was higher in the groups (3) and (4) involving the test subjects having a body weight (BW) of BW≤60 kg than in the groups (1) and (2) involving the test subjects having creatinine clearance (Ccr) of Ccr≤50.

Furthermore, the secondary fibrinolysis biomarker D-dimer, which has been reported to be increased in atrial fibrillation patients and decreased by anticoagulant therapy using warfarin, exhibited a numeric value comparable to that of warfarin on 28 days after the administration of the compound (1a). Moreover, even in test subjects having no history of warfarin administration, D-dimer exhibited a numeric value comparable to that of warfarin-administered patients on 28 days after the administration of compound (1a).

Industrial Applicability

An activated blood factor X (FXa) inhibitor of the present invention is useful as a therapeutic agent for thromboembolism that reduces the risk of bleeding in a patient.

## Claims

1. An oral anticoagulant agent comprising $N^1$-(5-chloropyridin-2-yl)-$N^2$-{(1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide represented by the following formula (1):

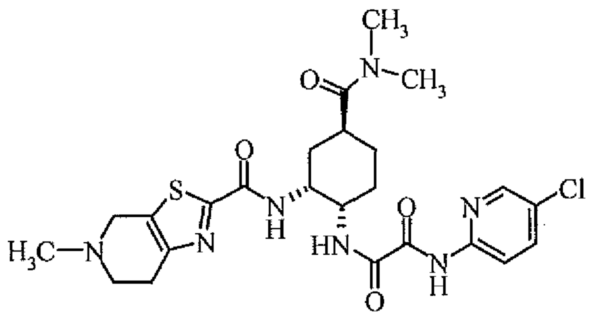

(1)

or a pharmacologically acceptable salt thereof, or a hydrate thereof, as an active ingredient, for use in the treatment of thromboembolism based on non-valvular atrial fibrillation wherein the dose of the anticoagulant agent is selected by

(A) selecting a factor involved in the risk of bleeding caused by the anticoagulant agent as a dose determinant, wherein the dose determinant of a patient in need of administration is creatinine clearance;
(B) setting a reference value of the dose determinant, wherein the reference value of the creatinine clearance is 50 ml/min;
(C) measuring the dose determinant of a patient in need of administration; and
(D) selecting the dose of the anticoagulant agent such that: (i) when the measured value is larger than the reference value, the dose is 30 to 60 mg/day in terms of the amount of the compound of formula (1); or (ii) when the measured value is equal to or smaller than the reference value, the dose is 1/2 that in the case where the measured value is larger than the reference value.

## Patentansprüche

1. Orales antikoagulierendes Mittel, umfassend $N^1$-(5-Chlorpyridin-2-yl)-$N^2$-{(1S,2R,4S)-4-[(Dimethylamino)carbonyl]-2-{[(5-methyl-4,5,6,7-tetrahydrothiazolo[5,4-c]pyridin-2-yl)carbonyl]amino}cyclohexyl)ethandiamid, dargestellt durch die folgende Formel (1):

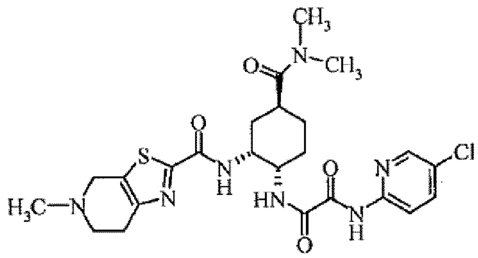

(1)

oder ein pharmakologisch verträgliches Salz davon, oder ein Hydrat davon, als Wirkstoff, zur Verwendung in der Behandlung von Thromboembolie, basierend auf nicht-valvulärem Vorhofflimmern, wobei die Dosis des antikoagulierenden Mittels ausgewählt wird durch

(A) Auswählen eines Faktors, beteiligt an dem Blutungsrisiko, verursacht durch das antikoagulierende Mittel, als einer Dosis-Determinante, wobei die Dosis-Determinante von einem Patienten, der der Verabreichung bedarf, Kreatinin-Clearance ist;
(B) Einstellen eines Referenzwerts der Dosis-Determinante, wobei der Referenzwert der Kreatinin-Clearance 50 ml/min beträgt;
(C) Messen der Dosis-Determinante von einem Patienten, der der Verabreichung bedarf; und
(D) Auswählen der Dosis des antikoagulierenden Mittels, derart, dass: (i), wenn der gemessene Wert größer als der Referenzwert ist, die Dosis 30 bis 60 mg/Tag hinsichtlich der Menge der Verbindung der Formel (1) beträgt; oder (ii), wenn der gemessene Wert gleich oder kleiner als der Referenzwert ist, die Dosis 1/2 von der in dem Fall, wo der gemessene Wert größer als der Referenzwert ist, beträgt.

**Revendications**

**1.** Agent anticoagulant oral comprenant le $N^1$-(5-chloropyridin-2-yl)-$N^2$-((1S,2R,4S)-4-[(diméthylamino)carbonyl]-2-{[(5-méthyl-4,5,6,7-tétrahydrothiazolo[5,4-c]pyridin-2-yl)carbonyl]amino}-cyclohexyl)éthanediamide représenté par la formule (1) suivante:

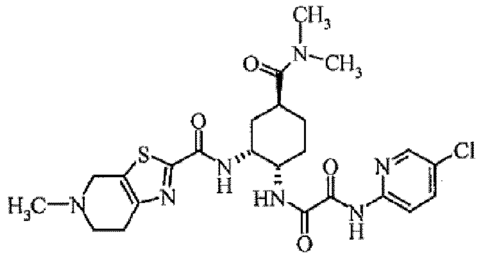
(1)

ou un sel pharmacologiquement acceptable de celui-ci, ou un hydrate de celui-ci, en tant qu'ingrédient actif, pour une utilisation dans le traitement d'une thrombo-embolie basée sur une fibrillation auriculaire non valvulaire, où la dose de l'agent anticoagulant est choisie en:

(A) choisissant un facteur impliqué dans le risque d'hémorragie entraîné par l'agent anticoagulant comme déterminant de dose, où le déterminant de dose d'un patient ayant besoin d'une administration est la clairance de créatinine;
(B) fixant une valeur de référence du déterminant de dose, où la valeur de référence de la clairance de créatinine est 50 ml/min;
(C) mesurant le déterminant de dose d'un patient ayant besoin d'une administration; et
(D) choisissant la dose de l'agent anticoagulant de sorte que: (i) lorsque la valeur mesurée est supérieure à la valeur de référence, la dose est de 30 à 60 mg/jour en termes de la quantité du composé de la formule (1); ou (ii) lorsque la valeur mesurée est inférieure ou égale à la valeur de référence, la dose est 1/2 de celle dans le cas où la valeur mesurée est supérieure à la valeur de référence.

[Figure 1]

| | 5 mg QD | 15 mg QD | 30 mg QD | 60 mg QD | Placebo |
|---|---|---|---|---|---|
| The number of cases | 88 | 92 | 88 | 88 | 89 |
| VTE incidence | 26/88 (29.5%) | 24/92 (26.1%) | 11/88 (12.5%) | 8/88 (9.1%) | 43/89 (48.3%) |
| 95% confidence interval | 20.0% – 39.1% | 17.1% – 35.1% | 5.6% – 19.4% | 3.1% – 15.1% | 37.9% – 58.7.% |
| P-value (vs. placebo) | 0.005 | <0.001 | <0.001 | <0.001 | |
| P-value [among compound (1a) groups] | <0.001 | | | | |

[Figure 2]

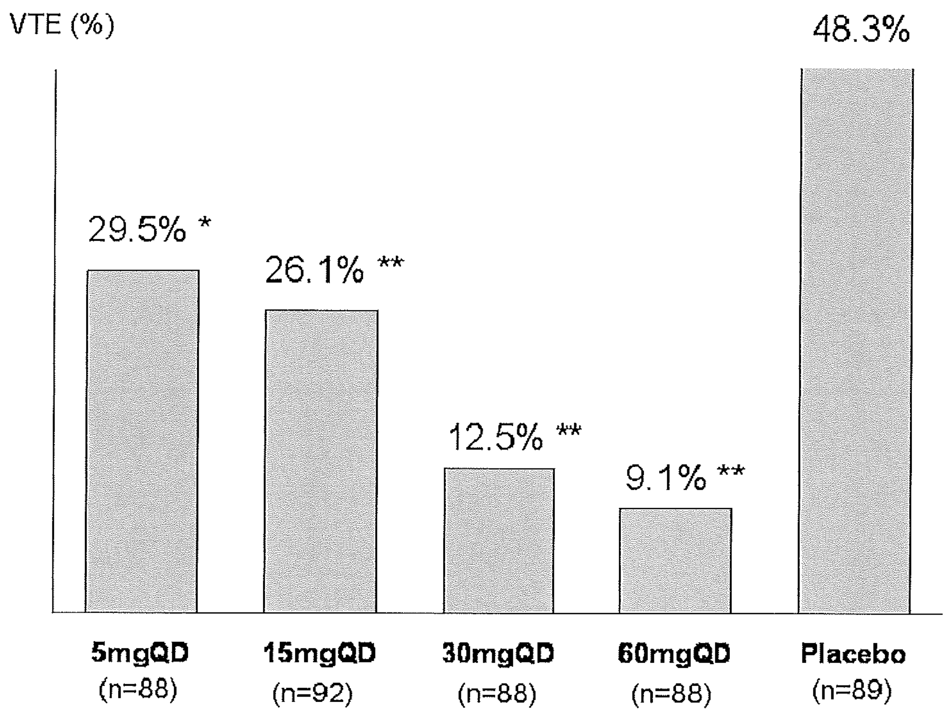


* p=0.005 (vs Placebo) ; ** p<0.001(vs Placebo) ; n: the number of cases

[Figure 3]

| Dose | 5 mg QD | 15 mg QD | 30 mg QD | 60 mg QD | Placebo |
|---|---|---|---|---|---|
| The number of cases | 103 | 106 | 103 | 106 | 102 |
| <Bleeding event><br>Major bleeding<br><br>Clinically relevant non-major bleeding<br><br>Subtotal | 0<br>(0%)<br>2<br>(1.9%)<br>2<br>(1.9%) | 0<br>(0%)<br>4<br>(3.8%)<br>4<br>(3.8%) | 0<br>(0%)<br>4<br>(3.9%)<br>4<br>(3.9%) | 1<br>(0.9%)<br>4<br>(3.8%)<br>5<br>(4.7%) | 0<br>(0%)<br>4<br>(3.9)<br>4<br>(3.9%) |
| P-value (vs. placebo) | 0.445 | 1.000 | 1.000 | 1.000 | |
| P-value [among compound (1a) groups] | 0.270 | | | | |
| Major bleeding event | 6<br>(5.8%) | 11<br>(10.4%) | 11<br>(10.7%) | 18<br>(17.0%) | 7<br>(6.9%) |
| P-value (vs. placebo) | 0.761 | 0.367 | 0.334 | 0.025 | |

[Figure 4]

| | | Warfarin (n=129) | Compound (1a) (n=396) | | | |
|---|---|---|---|---|---|---|
| | | | 30 mg QD (n=131) | 45 mg QD (n=134) | 60 mg QD (n=131) | |
| Age | | 69.0 | 69.0 | 70.5 | 69.0 | P=0.625 |
| Sex | male | 107 | 110 | 109 | 107 | P=0.941 |
| | Female | 22 | 21 | 25 | 24 | |
| Body weight (kg) | | 64.9 | 65.8 | 63.8 | 66.0 | P=0.303 |
| Height (cm) | | 162.8 | 163.3 | 162.6 | 163.1 | P=0.898 |
| BMI (kg/m²) | | 24.44 | 24.62 | 24.06 | 24.71 | P=0.321 |
| Risk factor | Hypertension (%) | 92(71) | 98(75) | 96(72) | 97(74) | P=0.897 |
| | Diabetes (%) | 40(31) | 24(18) | 28(21) | 28(21) | P=0.077 |
| | CHF (%) | 43(33) | 31(24) | 37(28) | 32(24) | P=0.285 |
| | History of Stroke/TIA (%) | 39(30) | 30(23) | 38(28) | 39(30) | P=0.528 |
| | Age>=75y (%) | 35(27) | 38(29) | 38(28) | 39(30) | P=0.971 |
| CHADS2 Score, mean | | 2.2 | 1.9 | 2.1 | 2.1 | P=0.153 |
| Warfarin Naive (%) | | 18(14) | 20(15) | 22(16) | 20(15) | P=0.958 |
| Current smoking use (%) | | 21(16) | 23(18) | 18(13) | 24(18) | P=0.817 |
| Current alcohol use (%) | | 84(65) | 85(65) | 80(60) | 74(57) | P=0.404 |
| CLCR, mL/min <50 (%) | | 16(12) | 13(10) | 21(16) | 16(12) | P=0.559 |

[Figure 5]

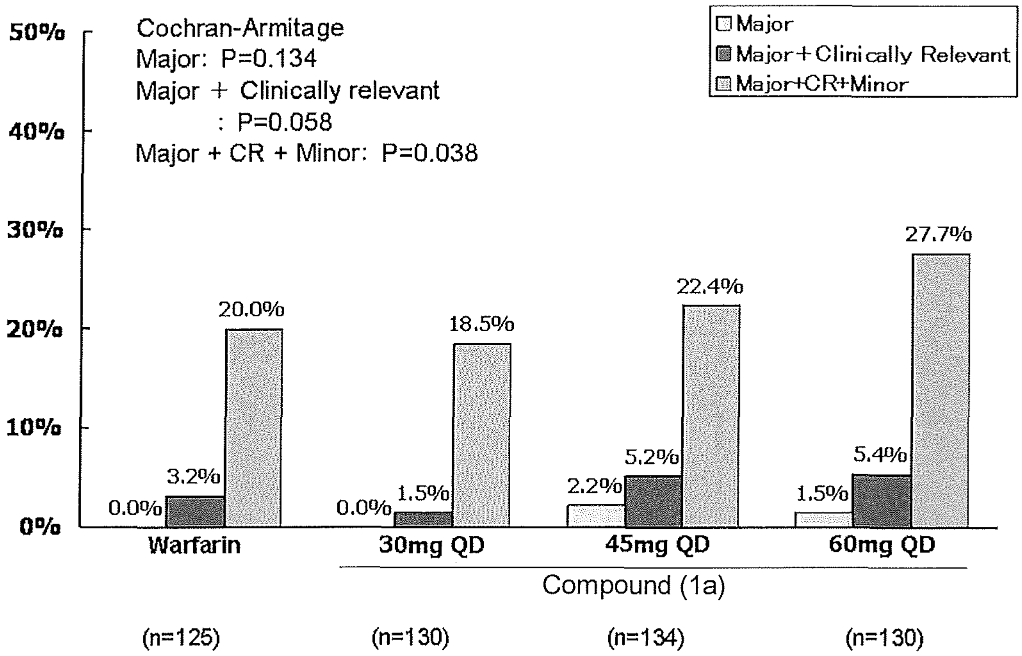
50%
40%
30%
20%
10%
0%
Cochran-Armitage
Major: P=0.134
Major + Clinically relevant
: P=0.058
Major + CR + Minor: P=0.038
Major
Major+Clinically Relevant
Major+CR+Minor
0.0%
3.2%
20.0%
0.0%
1.5%
18.5%
2.2%
5.2%
22.4%
1.5%
5.4%
27.7%
Warfarin
30mg QD
45mg QD
60mg QD
Compound (1a)
(n=125)
(n=130)
(n=134)
(n=130)

[Figure 6]

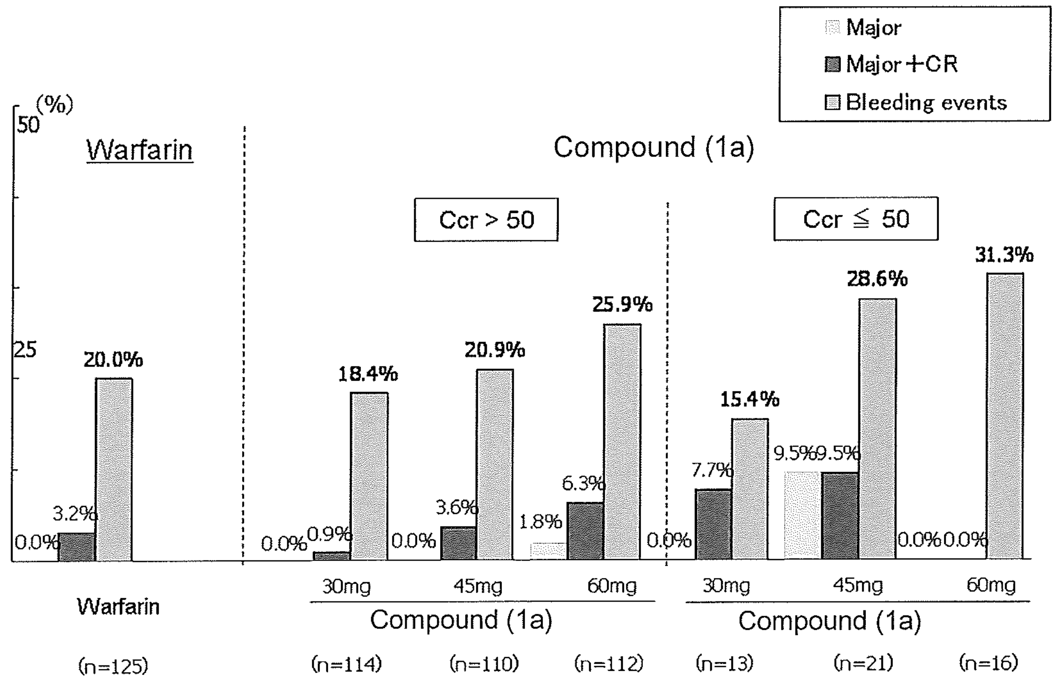


[Figure 7]

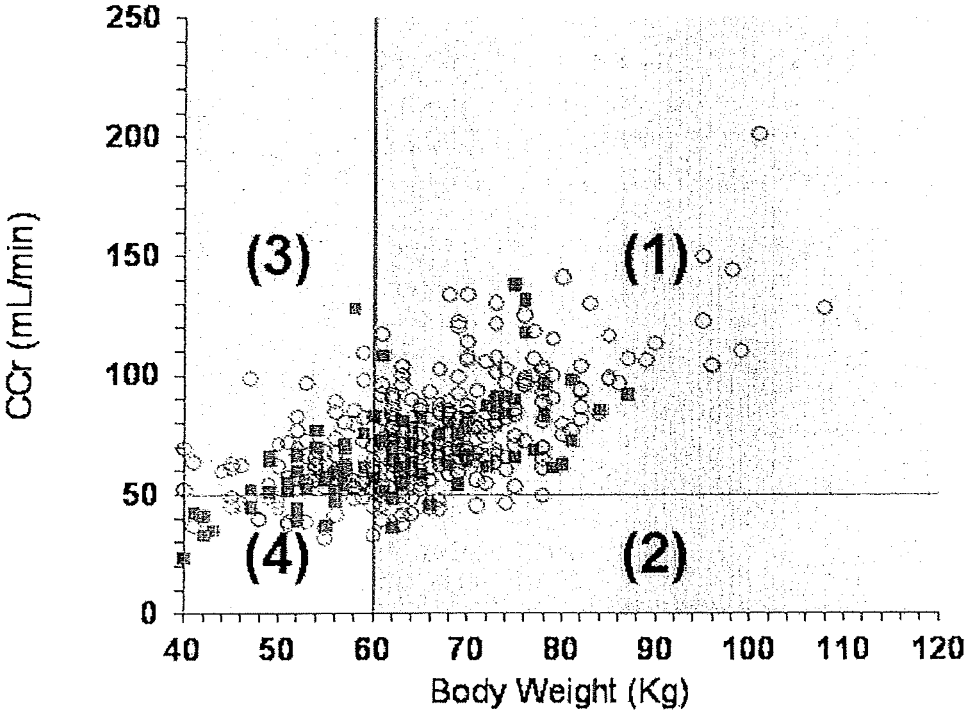

[Figure 8]

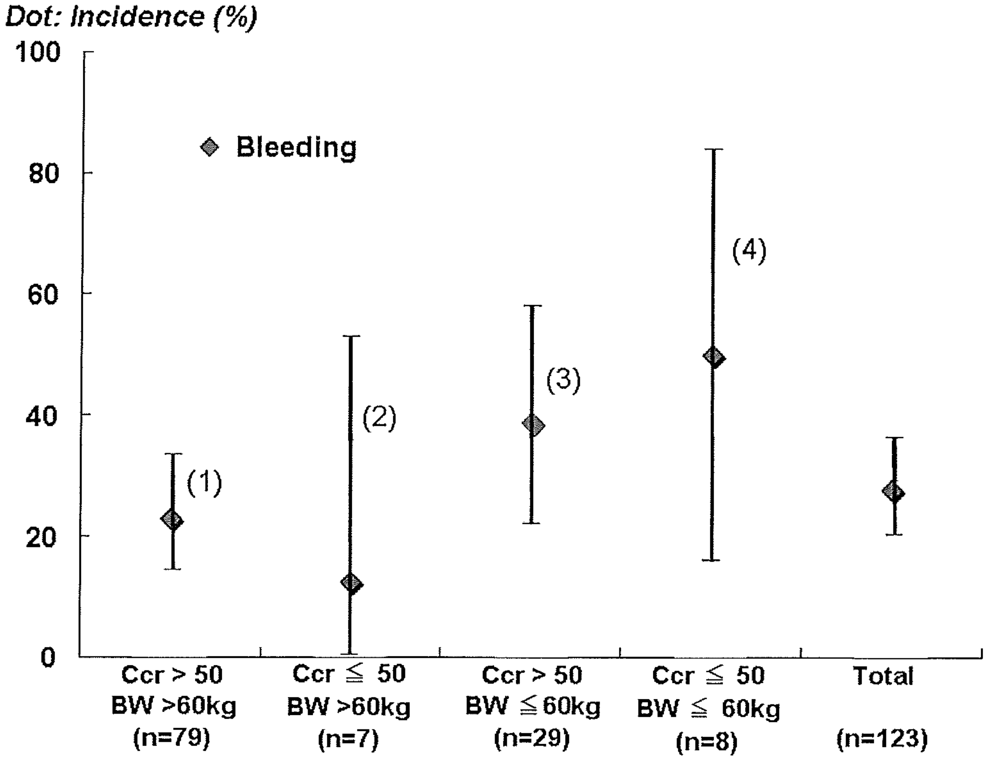
Dot: Incidence (%)
100
80
60
40
20
0
◆ Bleeding
(1)
(2)
(3)
(4)
Ccr > 50
BW >60kg
(n=79)
Ccr ≦ 50
BW >60kg
(n=7)
Ccr > 50
BW ≦60kg
(n=29)
Ccr ≦ 50
BW ≦ 60kg
(n=8)
Total
(n=123)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2003000680 A
- WO 2004058715 A


### Non-patent literature cited in the description


- **M. U. ZAFAR et al.** Thrombosis and Haemostasis. Schattauer GmbH, 01 October 2007, vol. 98, 883-888
- *Thrombosis Research,* 1979, vol. 15, 617-629
- *Thrombosis Research,* 1992, vol. 68, 507-512
- *PRODUCT INFORMATION, CLEXANE AND CLEXANE FORTE, CLEXANE PI MKT, #6178v16,* 1-19
- **FUJIMOTO et al.** *Japanese Journal of Nutrition,* 1968, vol. 28 (6
- Standards for the Implementation of Clinical Trials on Pharmaceutical Products. *80-2 of the Pharmaceutical Affairs Law and MHW Ordinance No. 28,* 27 March 1997
- **N. VENE et al.** *Thromb Haemost,* 2003, vol. 90, 1163-72
- Standards for the Implementation of Clinical Trials on Pharmaceutical Products. *Pharmaceutical Affairs Law and MHW Ordinance No. 28,* 27 March 1997